# EUROPEAN PATENT SPECIFICATION

(11) **EP 4 380 622 B1**
(45) Date of publication and mention of the grant of the patent: **16.04.2025**
(21) Application number: 22758239.2
(22) Date of filing: 02.08.2022
(51) Int. Cl.: A61K 47/26, A61K 47/32, A61K 47/36, A61K 47/38, A61K 9/00, A61K 9/20, A61K 9/16

(54) **METHOD FOR PREPARING CO-PROCESSED EXCIPIENT GRANULES**
VERFAHREN ZUR HERSTELLUNG VON CO-PROZESSIERTEN HILFSSTOFFGRANULATEN
PROCÉDÉ DE PRÉPARATION DE GRANULES D'EXCIPIENTS COTRAITÉS

(30) Priority: 03.08.2021 EP 21189255
(43) Date of publication of application: 12.06.2024
(73) Proprietor: DFE Pharma GmbH & Co. KG, 47574 Goch (DE)
(72) Inventor: HEBBINK, Gerrit Albert, 6708 WH Wageningen (NL); VAN HAANDEL, Mara Maria Wilhelmina, 6708 WH Wageningen (NL); JASPERS, Maarten, 6708 WH Wageningen (NL); VAN LAARHOVEN, Bas, 6708 WH Wageningen (NL); KULKARNI, Sri Sharath, 6708 WH Wageningen (NL)
(74) Representative: FrieslandCampina IP Department
(86) International application number: PCT/EP2022/071702
(87) International publication number: WO 2023/012162

(56) References cited:
- WO-A1-2008/020990
- WO-A2-2012/075455
- US-A1- 2018 214 835
- BIN LIEW KAI ET AL: "A REVIEW ON CO-PROCESSED EXCIPIENTS: CURRENT AND FUTURE TREND OF EXCIPIENT TECHNOLOGY", vol. 11, no. 1, 1 January 2019 (2019-01-01), IN, pages 1, XP055797870, ISSN: 0975-1491, Retrieved from the Internet <URL:https://innovareacademics.in/journals/index.php/ijpps/article/viewFile/29265/16530> DOI: 10.22159/ijpps.2019v11i1.29265

## Description

### FIELD OF THE INVENTION

The invention relates to a method for preparing co-processed excipient granules, to co-processed excipient granules obtainable by said method, and to the use of co-processed excipient granules obtainable according to the invention, in a tableting process.

### BACKGROUND TO THE INVENTION

In the pharmaceutical industry, the most frequently used method to administer an active pharmaceutical ingredient (abbreviated as "API") is a tablet. Such a tablet is made by compressing a mixture of one or more API's and one or more pharmaceutical excipients into a tablet. Several types of excipients can be distinguished, each having its own technical contribution in the preparation of tablets, but also during the disintegration of the tablet, for instance in water, or in the oral cavity. Different classes of excipients can be identified:
- Fillers, binders or diluents, which serve to provide a matrix that holds the tablet together after compressing, and to provide volume ("filling") to the tablet; examples are lactose, (microcrystalline) cellulose, mannitol, starches, sucrose, cellulose ethers, and dicalcium phosphate;
- Lubricants, which serve to prevent adherence of powders to equipment surfaces and promote tablet ejection from the die after compression; examples are magnesium stearate, talc, silica, magnesium stearyl fumarate;
- Superdisintegrants, that enable the tablet to disintegrate quickly when brought into contact with water or the saliva in the oral cavity; examples are sodium starch glycolate, croscarmellose sodium and cross-linked polyvinylpyrrolidone (crospovidon).

In the art, over the years, the use of excipient ingredients that contain more than one excipient, has gained importance. Such combinations can be made by simple mixing the individual excipients, but they are also available as so-called co-processed excipients, wherein the excipient mix has been subjected to a special process, for instance wet or dry granulation or agglomeration, which not only combines multiple excipients into one product but also improves its functional properties such as flow or compaction compared to a simple physical mixture. This makes such co-processed excipients particularly suitable for direct compression processes. Furthermore, co-processing of multiple excipients also prevents segregation of the individual components.

The pharmaceutical industry is, on the one hand, continuously seeking for improvements on excipient blends that have improved properties, such as good compressibility, quick release of an API when dissolved, or for instance as an ODT (orally disintegrating tablets) formulation.

On the other hand, the production of co-processed excipients needs also improvement. The majority of the current commercial co-processed excipients are prepared by a suspension of various individual constituents. The suspension is spray dried to obtain the co-processed granules. This process is essentially a continuous process but has the downside that suspensions require some form of atomization in the spray drier. Therefore the suspensions viscosity is limited in turn limiting the ratio of dissolved and undissolved constituents.

A batch-by-batch production process as used for instance in fluid bed granulation, may suffer from batch-to-batch variation, offer limited flexibility in batch size, and is cost-inefficient.

Furthermore, it is a challenge in the production processes of co-processed excipients that contain superdisintegrants, to keep the latter in physical active form, as understandably, these are very sensitive to moisture if that is used in some agglomeration and granulation processes.

High shear which is frequently used in granulation processes is also unfavorable because this can cause mechanical degradation of individual components resulting in a loss of functionality and stability. Furthermore, since high shear granulation is a batch-wise process, the scale of the process has a large impact on product properties.

Therefore, the need exists for an improved process for making a co-processed excipient that alleviates one or more of the above-mentioned drawbacks.

WO 2008/020990 shows a process in which an excipient physical blend is used in tableting process. A physical blend has the disadvantage that it may be prone to particle segregation. Furthermore the physical blending of excipients does not improve their flowability or other functional properties and therefore a granulation step may be required when the blend is combined with a poorly flowing API.

WO 2012/075455 describes a granulation process to prepare granulates of a sugar alcohol or saccharide, a superdisintegrant and a multifunctional additive.

US 10792634 provides for a process with a twin screw extruder wherein excipient granules are prepared using steam.

EP 3445337 B1 discloses a dry granulation process for producing solid formulations, using a twin screw extruder.

### SUMMARY OF THE INVENTION

It has now been uncovered that by modification of the twin screw extrusion production process of co-processed excipients, surprisingly an improved functionality of a co-processed excipient comprising at least one binder and at least one superdisintegrant was found. An important aspect of the invention resides in the fact that in the processing, the introduction of the binder and the superdisintegrant is done on different locations in the extruder that is used in the process.

Accordingly, in a first aspect, the invention relates to a method for preparing co-processed excipient granules using a co-rotating twin-screw extruder, the twin screw extruder having at least a conveying zone, a kneading zone and a mixing zone, the method comprising the steps of
a. Feeding at least one binder to the conveying zone of the extruder;
b. Conveying the binder towards the kneading zone;
c. Feeding a solvent into at least one point between the beginning and the end of the conveying zone of the extruder, thereby wetting the binder;
d. Kneading the wetted binder in the kneading zone of the extruder to form binder agglomerates;
e. Conveying the kneaded, wetted binder agglomerates to the mixing zone;
f. Feeding one or more superdisintegrants to the mixing zone, thereby forming agglomerates of binder and the one or more superdisintegrants;
g. Collecting the agglomerates of binder and superdisintegrant from the extruder; and
h. Drying the agglomerates of binder and superdisintegrant to form the co-processed excipient granules;

wherein the binder is selected from the group consisting of
   (i) disaccharides;
   (ii) monosaccharides;
   (iii) polyols;
   (iv) cellulose derivatives selected from the group consisting of cellulose fibers, microcrystalline cellulose, carboxymethylcellulose, hydroxypropyl methyl cellulose, hydroxypropyl cellulose, and mixtures thereof;
   (v) starches;
   (vi) inorganic salts;
   and mixtures thereof;
and wherein the superdisintegrant is selected from the group consisting of sodium starch glycolate, croscarmellose sodium, crospovidon, low-substituted hydroxypropyl cellulose, soy polysaccharides, polacrilin potassium, calcium alginate, alginic acid, and mixtures thereof.

In a second aspect, the invention relates to co-processed excipient granules obtainable by the method of the invention.

In a third aspect, the invention relates to the use of co-processed excipient granules obtained by the method according to the invention in a tableting process.

### DETAILED DESCRIPTION

### Definitions.

"API" has its conventional meaning in the art and refers to an active pharmaceutical ingredient.

"Direct compression" has its conventional meaning in the art and refers to a tableting process in which a mixture of an API and suitable excipients is compressed directly without any preceding wet or dry granulation.

A "binder" has its conventional meaning in the art and refers to an excipient that holds the formulation of for example a tablet together. A binder may also provide volume to certain low active dosage tablets.

A "superdisintegrant" has its conventional meaning in the art and refers to an excipient that facilitates the disintegration of a tablet in a solvent or the oral cavity, and ensures a rapid dissolution of the tablet components.

"ODT" is orally disintegrating tablet and has its conventional meaning in the art and refers to tablets that are made to disintegrate quickly in the mouth.

A "co-rotating twin screw extruder" has its conventional meaning in the art and refers to an extruder in which two intermeshing screws rotate in the same direction.

**Conveying** elements: Screw shaped elements which are designed to transport the material through the extruder while imparting low shear forces on the material. The screw pitch determines the conveying capacity, where a longer pitch can accommodate a higher throughput.

Kneading zone /kneading elements: The kneading zone is made up of individual kneading elements which apply high mechanical energy on the wetted powder mass. Pairs of kneading elements provide a region of compaction in the intermeshing region of the screws. The compaction between kneading elements causes densification and squeezes liquid to the outside of the granule allowing for growth through consolidation. The kneading elements are typically offset at angles of 30°, 60° or 90°.

Mixer elements: Different types of distributive mixer elements are commonly used in a twin screw extrusion process. Mixer elements distribute and recombine flow streams in the extruder at low shear forces, providing both mixing and conveying of the materials. The mechanical energy applied to the material by the comb mixer elements is much lower compared to the kneading elements. Examples of mixer elements include comb mixer elements, screw mixer elements, toothed mixer elements and other distributive mixing elements.

Comb mixer elements are composed of rings arranged perpendicular to the direction of flow, containing angular cuts to allow for the passage of material through the element. Screw mixer elements consist of a standard screw profile with slots cut across the flight tip to increase leakage flow, which provides distributive mixing of material streams within the extruder. Screw mixer elements also result in backflow of material, resulting in increased residence time.

The term "wetting" or "wetted" relates to the process of contacting the binder particles with a solvent in general, which means that the term "wetting" is not restricted to wetting by only water, but includes contacting the binder with an organic solvent, with a mixture of organic solvents, or with a mixture of water and one or more organic solvents.

### Methods.

- Disintegration test definition:
   ∘ Disintegration time was measured on an Erweka disintegration tester (ZT122, Germany) with demineralized water at a temperature of 37 °C as medium. Disintegration time was reported in seconds when **the tablet** is dissolved. Measurements were performed on six tablets and the value is reported as the average of the six tablets.

- Staining method of the co-processed excipient granules:
   ∘ The granules were wetted with a mixture of Lugol solution (Iodine/Potassium Iodine in water) and glycerol. Subsequently the granules were observed under a stereomicroscope.

The present invention relates to an improved method for producing co-processed excipient granules, that provides better functionality with respect to disintegration time of tablets pressed with the granules prepared according to the invention. Furthermore, the process of the invention can be executed continuously, preferably in a steady state, in a confined space, and optionally with more than one extruder operating simultaneously, making it a highly efficient and scalable process. Additionally, the co-processed excipient granules prepared with the method of the invention allow for a high consistency and repeatability with respect to the composition and functionality of the excipient granules. A continuous process also allows the use of in-line process sensors to monitor and control product quality. In case of defects the affected product stream can be rejected. Once an adequate product quality has been achieved again- the commercial production is continued.

Accordingly, in a first aspect, the invention relates to a method for preparing co-processed excipient granules using a co-rotating twin-screw extruder, the twin screw extruder having at least a conveying zone, a kneading zone and a mixing zone, the method comprising the steps of
a. Feeding at least one binder to the conveying zone of the extruder;
b. Conveying the binder towards the kneading zone;
c. Feeding a solvent into at least one point between the beginning and the end of the conveying zone of the extruder, thereby wetting the binder;
d. Kneading the wetted binder in the kneading zone of the extruder to form binder agglomerates;
e. Conveying the kneaded, wetted binder agglomerates to the mixing zone;
f. Feeding one or more superdisintegrants to the mixing zone, thereby forming agglomerates of binder and the one or more superdisintegrants;
g. Collecting the agglomerates of binder and superdisintegrant from the extruder; and
h. Drying the agglomerates of binder and superdisintegrant to form the co-processed excipient granules;

wherein the binder is selected from the group consisting of (i) disaccharides; (ii) monosaccharides; (iii) polyols; (iv) cellulose derivatives selected from the group consisting of cellulose fibers, microcrystalline cellulose, carboxymethylcellulose, hydroxypropyl methyl cellulose, hydroxypropyl cellulose, and mixtures thereof; (v) starches; (vi) inorganic salts; and mixtures thereof;
and wherein the superdisintegrant is selected from the group consisting of sodium starch glycolate, croscarmellose sodium, crospovidon, low-substituted hydroxypropyl cellulose, soy non-starch polysaccharides, polacrilin potassium, calcium alginate, alginic acid, and mixtures thereof.

The skilled person will be aware that in the process according to the invention, the mixing zone is downstream of the kneading zone.

The co-rotating twin screw extruder used in the present invention is an apparatus known in the art and the skilled person is aware of the mode of operation of such a device. They are usually configured as modular machines, which means that certain parts of the screw configuration can be exchanged. The screws are preferably mounted on a splined shaft, preferably in a closed barrel.

The conveying zone, the kneading zone and the mixing zone of the extruder are comprised in a barrel, and preferably the length of the barrel is equal to the length of the above-mentioned zones taken together. Preferably, the length-to-diameter ratio (L/D) of the barrel is between 20 and 40.

An example of the general configuration of a co-rotating twin screw extruder including the technical terms used for such extruders is depicted in https://extruders.leistritz.com/en-row/extrusion/brochures/leistritz-zse-maxx-en.pdf.

In the method of the present invention, the extruder comprises at least a conveying zone which preferably comprises two intermeshing, co-rotating screws. One or more screw units may be installed on the shaft to adjust the length of the conveying zone. The conveying zone is preferably used to transport the at least one binder towards the kneading zone.

The binder may preferably be fed into the extruder with a volumetric or gravimetric feeder at constant feed rate. The binder feed rate can be varied to increase or decrease the throughput of the process.

The kneading zone of the extruder preferably comprises kneading elements, preferably 3-10 kneading elements. Preferably, the angle of offset of the kneading elements is in the range of 30°-90°.

The kneading zone serves to agglomerate the binder in the presence of the solvent, added to the binder at a point before the kneading element.

The solvent used in the method of the invention may be selected from the group consisting of water, an organic solvent, and mixtures thereof.

The preferred solvent is water.

In another embodiment, preferably, the solvent is an organic solvent, more preferably chosen from the group of ethanol, acetone, n-propanol, isopropanol, and mixtures thereof. Most preferred is isopropanol as organic solvent.

The kneading elements apply high shear forces to the binder when it passes through and they also provide mixing of the binder and the solvent. The combination of mixing with solvent and high shear force results in granulation of the binder particles into larger binder agglomerates.

Preferably, a second conveying zone may be present in the extruder located between the kneading zone and the mixing zone. This second conveying zone serves to transport the binder agglomerates formed in the kneading element towards the mixing zone.

The mixing zone of the co-rotating twin screw extruder preferably comprises at least one mixer element, more preferably a comb mixer element or a screw mixer element.

In the mixing zone, one or more superdisintegrants are added to the formed binder agglomerates, which agglomerates preferably have been conveyed from the kneading zone to the mixing zone through the second conveying zone.

The at least one mixer element preferably provides distributive mixing of the binder and superdisintegrant.

In the mixing zone, agglomerates of binder and superdisintegrants are formed. It is has been found that in the mixing zone, the superdisintegrant starts adhering to the surface of the binder agglomerates.

Preferably, in an embodiment, a third conveying zone comprising conveying elements such as intermeshing co-rotating screws is present in the extruder, located after the mixing zone, wherein conveying elements transport the agglomerates of binder and superdisintegrant to the outlet of the extruder.

The screw speed of the extruder is preferably kept constant, more preferably constant at a rate between 100 - 1000 rpm.

Preferably, the angular velocity of the screw is set at a value between 1 - 42 rad/s, more preferably 15 - 37 rad/s, most preferably 21 - 31 rad/s.

The skilled person is aware how to set the screw speed and/or the angular velocity of the screw.

It has been found that steps a. - g. can be executed at relatively low temperatures, making the method energy-efficient, and causes minimal damage to the binder and superdisintegrant. Hence, the temperature in steps a. - g. is preferably kept between 10°C - 60°C, more preferably between 15°C - 50°C, most preferably between 20°C - 30°C.

Preferably, the agglomerates of binder and superdisintegrant are collected from the outlet of the extruder.

Drying of the agglomerates of binder and superdisintegrant to form the co-processed excipient granules preferably takes place in a suitable drier, more preferably in fluid bed drier. Preferably, the drying takes place for at least 5 minutes, more preferably for between 5 - 10 minutes. The drying temperature preferably lies between 40°C and 80°C.

Preferably, the co-processed excipient granules are further subjected to a milling and/or sieving step, to control the particle size distribution of the granules. More preferably, the sieving step is done to remove too large granules, for instance larger than 600 µm.

Preferably, the milling step is done to break down too large particles, for instance to reduce the average granule size to less than 200 µm.

The milling step may preferably be followed by a sieving step.

It is preferred that the median particle size of the co-processed excipient granules, defined as x50, lies between 100-200 µm.

The median particle size, defined as x50, is the size at which half of the particles are larger and the other half are smaller as determined via laser diffraction.

It is preferred that the binder is in solid state at about 25°C. The binder is more preferably a powder or a granulate.

As already indicated, the binder according to the invention is selected from the group consisting of (i) disaccharides; (ii) monosaccharides; (iii) polyols; (iv) cellulose derivatives selected from the group consisting of cellulose fibers, microcrystalline cellulose, carboxymethylcellulose, hydroxypropyl methyl cellulose, hydroxypropyl cellulose, and mixtures thereof; (v) starches; (iv) inorganic salts; and mixtures thereof. In a preferred embodiment, the binder comprises a disaccharide.

In an even more preferred embodiment, the binder is a disaccharide selected from the group consisting of lactose, sucrose, maltose, and mixtures thereof.

Most preferably, the disaccharide comprises lactose. It has been found that the use of lactose provides good results with respect to dissolving properties of tablets, compressed with the co-processed excipient granules according to the invention. If lactose is used, any type of powder form lactose may be used. For example, milled lactose types with a median particle size (x50) of 40 and 30 micron, as measured by laser diffraction measurements, may be used, but other types or grades can be used as well. A suitable laser diffraction instrument is a Sympatec Helos R, equipped with a Podos dry powder dispersion unit.

Most preferably, the lactose used is lactose monohydrate.

A suitable type of lactose monohydrate is f.i. Pharmatose 200M, Pharmatose 350M, or Lactochem Fine Powder from DFE Pharma, Germany.

In another embodiment, the binder is a monosaccharide, preferably it is glucose. In yet another embodiment, the binder is a polyol, preferably it is a polyol selected from the group consisting of mannitol, sorbitol, erythritol, xylitol, and mixtures thereof. Most preferred polyol is mannitol. A suitable mannitol may be obtained from Roquette or SPI Pharma, for instance Pearlitol 50C from Poquette.

In yet another embodiment, the binder is a cellulose derivative, selected from the group consisting of cellulose fibers, microcrystalline cellulose, carboxymethylcellulose, hydroxypropyl methyl cellulose, hydroxypropyl cellulose, and mixtures thereof. In an even more preferred embodiment, the cellulose derivative is microcrystalline cellulose, most preferred is microcrystalline cellulose with an x50 between 50µm and 100µm. Suitable types are Pharmacel 101 and Pharmacel 102, both from DFE Pharma, Germany.

Preferred starches as binder are chosen from the group consisting of native starch, partly gelatinized starch, fully pregelatinized starch, and mixtures thereof.

Starches are preferably derived from maize, potato or rice, and mixtures thereof. If the binder is an inorganic salt, it preferably is dicalcium phosphate.

The superdisintegrant used in the method of the invention is selected from the group consisting of sodium starch glycolate, croscarmellose sodium, crospovidon, low-substituted hydroxypropyl cellulose, soy non-starch polysaccharides, polacrilin potassium, calcium alginate, alginic acid, and mixtures thereof. Preferred is sodium starch glycolate or croscarmellose sodium or a combination of these two. Most preferred is sodium starch glycolate.

A suitable sodium starch glycolate is Primojel from DFE Pharma, Germany; a suitable croscarmellose sodium is Primellose from DFE Pharma, Germany; a suitable crospovidon is Polyplasdone XL from Ashland, USA.

For sake of clarity, with respect to the present invention, it must be understood that a binder and a superdisintegrant are not the same compounds.

Thus, it will therefore be clear to the skilled person with regard to the embodiments with hydroxypropyl cellulose as binder and *low-substituted* hydroxypropyl cellulose as superdisintegrant, that these two compounds are chemically different components, which differ from one another in their degree of substitution with hydroxypropyl groups.

Hydroxypropyl cellulose, which is suitable for use as a binder according to the present invention, is partly O-(2-hydroxypropylated) cellulose which is characterized by a content of not less than 53.4 % and not more than 80.5 % of hydroxypropyl groups (-OCH₂CHOHCH₃), calculated on dry basis, as defined in US Pharmacopoeial Convention stage 6 Harmonization, Official December 2014.

The analysis method of the hydroxypropyl content of HPC is also described in said USP document.

Low-substituted hydroxypropyl cellulose, which is suitable for use as a superdisintegrant according to the present invention, is a low-substituted O-(2-hydroxypropylated) cellulose characterized by a content of not less than 5.0 % and not more than 16.0 % of hydroxypropyl groups (-OCH₂CHOHCH₃), calculated on dry basis, as defined in US Pharmacopoeial Convention, stage 4 harmonization, Official May 1, 2019.

The analysis method of the hydroxypropyl content of low-substituted HPC is also described in said USP document.

For a good performance of the co-processed excipient granule, the wt./wt. ratio of the binder to superdisintegrant is important. Hence, the wt./wt. ratio of binder to superdisintegrant in the process is preferably between 99/1 and 80/20, more preferably between 99/1 and 90/10 , most preferably between 98/2 and 94/6.

It will be understood by the skilled person that the feed rate of the binder and the superdisintegrant to the applicable extruder zones are set in such a way that the desired wt. ratio of binder to superdisintegrant in the co-processed excipient granules is obtained.

In order to granulate the binder and thus form granules of binder, in the kneading step, an amount of solvent may be added to the binder in the process step c. Accordingly, in the method of the invention, the wt./wt. ratio of the solvent to binder is preferably between 1/100 and 50/100, more preferably between 3/100 - 30/100, most preferably between 5/100 - 25/100.

It is preferred that in the preparation method, the solvent is added in step c. in a controlled way, preferably using a metered dosing system and/or a pump, into the conveying zone, where it is brought into contact with the binder.

As described above, the solvent is fed into at least one point between the beginning and the end of the conveying zone of the extruder, thereby wetting the binder. In one embodiment, together with said solvent, a binder is added. In that case, preferably, the binder used in step c. is the same binder as used in step a. In another embodiment according to the invention, the binder used in step c. is a different binder as the one used in step a.

The invention further pertains to co-processed excipient granules obtainable by the method of the invention.

The co-processed excipient granules obtainable by the method of the invention preferably comprise at least one binder selected from the group consisting of (i) disaccharides; (ii) monosaccharides; (iii) polyols; (iv) cellulose derivatives selected from the group consisting of cellulose fibers, microcrystalline cellulose, carboxymethylcellulose, hydroxypropyl methyl cellulose, hydroxypropyl cellulose, and mixtures thereof; (v) starches; (vi) inorganic salts; and mixtures thereof, and a superdisintegrant selected from the group consisting of sodium starch glycolate, croscarmellose sodium, crospovidon, low-substituted hydroxypropyl cellulose, soy non-starch polysaccharides, polacrilin potassium, calcium alginate, alginic acid, and mixtures thereof. In a preferred embodiment, the binder is or comprises a disaccharide, preferably lactose. In another embodiment, the binder is or comprises a cellulose derivative selected from the group consisting of cellulose fibers, microcrystalline cellulose, carboxymethylcellulose, hydroxypropyl methyl cellulose, hydroxypropyl cellulose, and mixtures thereof, more preferably the binder is or comprises microcrystalline cellulose. In another preferred embodiment, the preferred superdisintegrant is sodium starch glycolate or croscarmellose sodium or a combination of these two. Most preferred is sodium starch glycolate.

The invention also pertains to the use of co-processed excipient granules obtained or obtainable by the method of the invention, in a tableting process. Preferably, the tableting process is a direct compression process. It is preferred that in the tableting process at least one active pharmaceutical ingredient is used. Preferably, in an embodiment, the tableting process is a process for preparing orally disintegrating tablets.

Further components, amounts and/or embodiments of the co-processed excipient granules of the invention are the same as those used in the method of the invention.

By virtue of its physical characteristics, and observed disintegration properties when applied in a tablet, the granules according to the invention are novel and different and perform better than co-processed excipients that are made using a one-step extrusion process, or a fluidized bed agglomeration process. In the granules according to the invention, the superdisintegrant is predominantly bound to the surface of the granules, which is in contrast to the one-step extrusion process wherein the binder and superdisintegrant are combined upfront and processed together from the beginning of the extrusion process, or a fluidized bed agglomeration process. In both these prior art methods, the superdisintegrant is trapped within the granule. This difference can e.g. when the disintegrant comprises sodium starch glycolate, be verified by using an iodine staining method that causes the superdisintegrant to assume a purple color. As a result, in the granules of the invention, using a microscope, discrete dark purple spots are visible on the surface of the co-processed excipient granule. On the other hand, the color in the excipient granules made by the other two methods shows a diffuse purple haze, indicating that the superdisintegrant is distributed evenly inside the granule, and not present as discrete spots on the surface of the granules.

Alternatively, the granules can be analyzed using Scanning Electron Microscope (SEM) technology.

### DESCRIPTION OF FIGURES

Fig. 1. Schematic drawing of twin screw extrusion setup used for making co-processed lactose-superdisintegrant products.
   1 = Lactose powder feeder, 2 = peristaltic pump, 3 = MilliQ purified water, 4 = Superdisintegrant addition point, 5 = conveying elements, 6 = kneading elements, 7 = Barrel temperature control, 8 = product outlet.
Fig. 2. Photograph of the screw configuration used in the extruder, indicating the different screw zones and the addition points of the three components.
Fig. 3. Microscopic images of iodine- stained co-processed excipients according to the invention. The superdisintegrant (Primojel) has been stained with iodine resulting in a purple color. The images show a lactose granule of several 100's of micrometers with Primojel particles attached to the surface
Fig. 4. Microscopic images of granulated lactose (Pharmatose 350M) with 4% superdisintegrant (Primojel) incorporated intragranulary. Iodine staining of Primojel shows that the superdisintegrant is incorporated homogeneously inside the granules. Scale bars represent 1 mm (top image) and 200 µm (bottom image). The iodine-stained co-processed excipients were made by twin screw extrusion whereby the binder and superdisintegrant were first mixed and then subjected to screw extrusion (not according to the invention).

### EXPERIMENTAL SECTION

### Example 1.

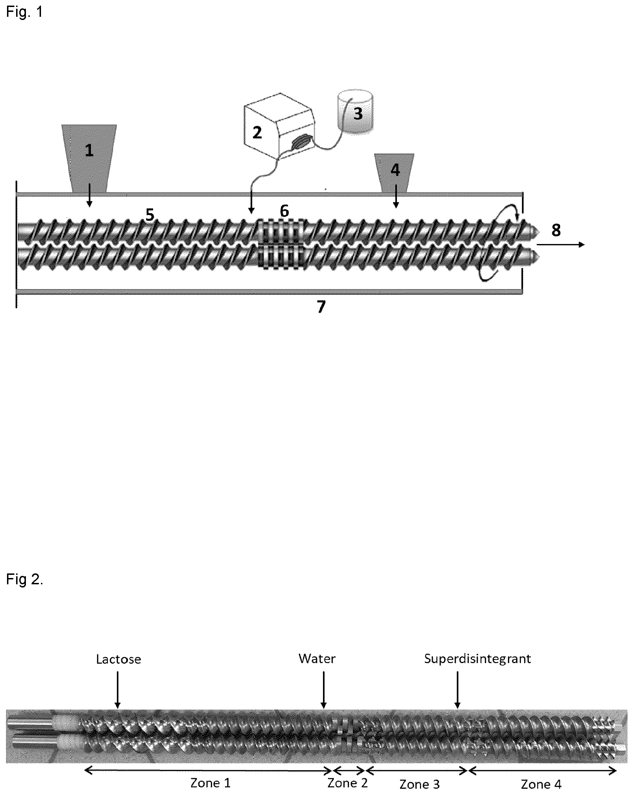

Preparation of co-processed excipient granules of lactose and superdisintegrant, according to the invention. See also fig. 1. Granulation was performed on a co-rotating twin screw extruder (Length : Diameter=25:1) (Eurolab 16, Thermo Fisher Scientific). Lactose monohydrate powder (the binder) was fed into the conveying zone of the extruder with a volumetric feeder at constant feed rate. The lactose feed rate can be varied to increase or decrease the throughput of the process. The lactose grades used were Pharmatose 200M and Pharmatose 350M (all from DFE Pharma), with average particle sizes of 40 um and 30 um respectively. The particle size of the lactose used as starting material has no significant impact on the final product, so finer or coarser lactose grades can also be used. The aqueous solvent (Milli-Q water at room temperature) was fed into the conveying zone but just before the kneading zone of the twin screw extruder through a second opening using a peristaltic pump (Sci-Q 300, Watson-Marlow, UK). The water/lactose wt./wt. ratio can be varied by controlling the speed of the pump, where a higher amount of water results in larger granules. The water/lactose wt./wt. ratio used in the current experiments is 1/10. The superdisintegrant was added into the mixing zone of the extruder manually, through a third opening. The amount of superdisintegrant can be varied to make products with different lactose/superdisintegrant ratios. In the current experiments, the amount of superdisintegrant was fixed at 4 wt.% of the amount of lactose used. The screw speed and barrel temperature of the extruder were kept constant at 250 rpm and 20 °C respectively.

The screw configuration used for granulation in the extruder consisted of four zones. The first zone consisted of conveying elements only. These conveying elements were used to transport the lactose from the feeder to the second opening in the extruder, where the water was added. The second zone of the screw configuration consisted of 5 kneading elements, which were staggered at a 60° forward angle and positioned directly after the point where the water was added into the extruder. The kneading elements applied high shear forces to the powder when it passed through and they also provided mixing of the lactose powder and the water. The combination of mixing with water and high shear force resulted in granulation of the lactose particles into larger lactose granules. The third zone of the screw configuration again consisted of conveying elements that were used to transport the material to the third port of the extruder where the superdisintegrant is added. After the point of superdisintegrant addition, the fourth zone of the screw configuration consisted of a combination of conveying elements and comb mixer elements. These comb mixer elements provided distributive mixing of the lactose and superdisintegrant and the conveying elements were used to transport the final product to the outlet of the extruder, where the product was collected. After collection, the product was dried in a fluid bed dryer for 5-10 minutes at a temperature of 60 °C to remove the water. To control the particle size of the final product, it was milled to break down the over-sized granules. The median particle size (x50) of the final product was about 200 µm.

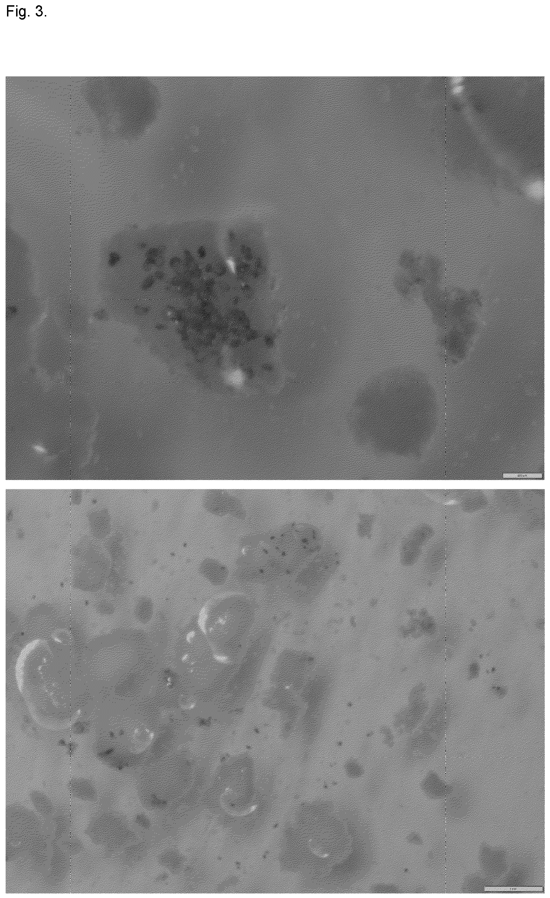

The granules were iodine-stained, see figure 3. Clearly visible are the superdisintegrant spots on the outside of the granules.

### Comparative Example 2.

Preparation of co-processed excipient granules of lactose and superdisintegrant, not according to the invention.

Lactose (Pharmatose 350M) and sodium starch glycolate (Primojel) were preblended in 2 liter jar using a Turbula blender at 96 rpm for 10 minutes. The amount of superdisintegrant was fixed at 4 wt.% of the amount of lactose used. The resulting blend was fed into the conveying zone of the extruder with a volumetric feeder at constant feed rate. The aqueous solvent (Milli-Q water at room temperature) was fed into the conveying zone but just before the kneading zone of the twin screw extruder through a second opening using a peristaltic pump (Sci-Q 300, Watson-Marlow, UK). The water/lactose wt./wt. ratio used in the current experiment is 1/10. The screw speed and barrel temperature of the extruder were kept constant at 250 rpm and 20 °C respectively. The screw configuration used is the same as in example 1. The product is collect from the extruder and subsequently dried in a fluid bed dryer for 5-10 minutes at a temperature of 60 °C to remove the water. To control the particle size of the final product, it was milled to break down the over-sized granules. The median particle size (x50) of the final product was about 200 µm.

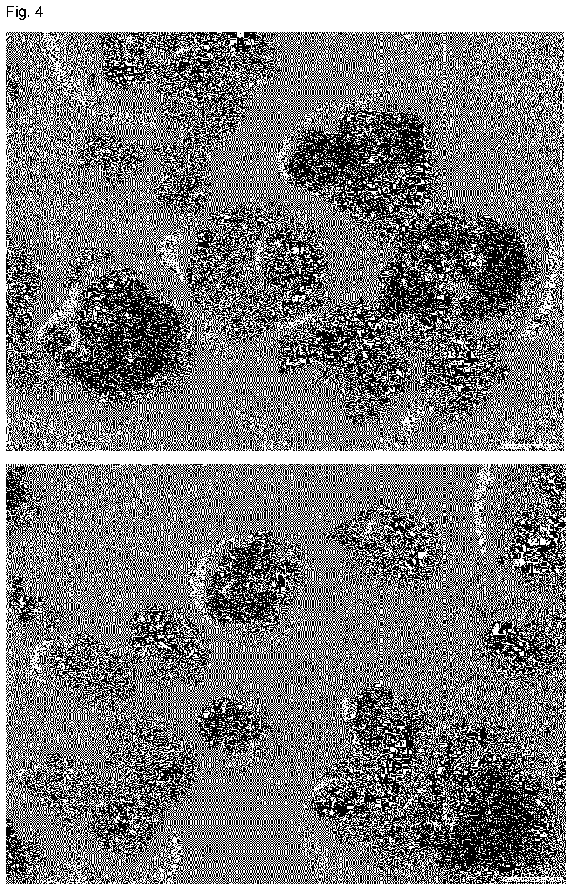

These granules were also iodine-stained (fig. 4) and very clearly the particles show a diffuse purple haze, indicating that the superdisintegrant was evenly distributed inside the granules.

### Example 3.

Preparation of ODT tablets with the samples from example 1 and 2.

Prior to tableting, all materials were conditioned in a climate chamber at a temperature of 20°C and a relative humidity of 30% overnight (HPP110, Memmert). Subsequently, 0.5% w/w of magnesium stearate was added and blended in a Turbula blender at 96 rpm for 2 minutes.

Tablets were compressed with the rotary tablet press (PoTab T, Luxner) using a rotating frequency of 25 rpm and a turret speed of 13 rpm, resulting in a dwell times of 60 ms. The filling depth of the die was adjusted to obtain tablets of 250 (±2) mg each. The punches used were flat beveled with diameter of 9 mm (iHolland). The tablets were compacted at three different compression forces of 5 kN, 10 kN, and 15 kN.

### Example 4.

Tablets were analyzed by an automated tablet tester (Sotax AT50) for their weight, diameter, thickness, and crushing strength. The tablet crushing strength is the maximum force required to break the tablets and this force was measured at a constant speed of 120 mm/min. The tablet tensile strength (TTS) was calculated from the tablet crushing strength, diameter, and thickness of the tablets. The values reported are an average of twenty tablets.

Disintegration time was measured on an Erweka disintegration tester (ZT122, Germany) with demineralized water at a temperature of 37 °C as medium. Disintegration time was reported in seconds when the tablet is dissolved. Measurements were performed on six tablets and the value is reported as the average of the six tablets.

### Tablet tensile strength results:

| **Compression force** | **Example 1** | **Comparative Example 2** |
|---|---|---|
| 5 kN | 0.49 MPa | 0.70 MPa |
| 10 kN | 1.31 MPa | 1.62 MPa |
| 15 kN | 2.04 MPa | 2.78 MPa |

### Disintegration time results (seconds):

| **Compression force** | **Example 1** | **Comparative Example 2** |
|---|---|---|
| 5 kN | 31 s | 1632 s |
| 10 kN | 46 s | 1648 s |
| 15 kN | 46 s | 1662 s |

The results show that the tablets made using the co-processed excipient granules according to the invention (Example 1) disintegrate much faster than the tablets made with the reference co-processed excipient granules (Comparative Example 2), whereas the tensile strength values at 10 and 15 kN compression force are sufficient.

## Claims

1. Method for preparing co-processed excipient granules using a co-rotating twin-screw extruder, the twin screw extruder having at least a conveying zone, a kneading zone and a mixing zone, the method comprising the steps of
a. Feeding at least one binder to the conveying zone of the extruder;
b. Conveying the binder towards the kneading zone;
c. Feeding a solvent into at least one point between the beginning and the end of the conveying zone of the extruder, thereby wetting the binder;
d. Kneading the wetted binder in the kneading zone of the extruder to form binder agglomerates;
e. Conveying the kneaded, wetted binder agglomerates to the mixing zone;
f. Feeding one or more superdisintegrants to the mixing zone, thereby forming agglomerates of binder and the one or more superdisintegrants;
g. Collecting the agglomerates of binder and superdisintegrant from the extruder; and
h. Drying the agglomerates of binder and superdisintegrant to form the co-processed excipient granules;
wherein the binder is selected from the group consisting of
(i) disaccharides;
(ii) monosaccharides;
(iii) polyols;
(iv) cellulose derivatives selected from the group consisting of cellulose fibers, microcrystalline cellulose, carboxymethylcellulose, hydroxypropyl methyl cellulose, hydroxypropyl cellulose, and mixtures thereof;
(v) starches;
(vi) inorganic salts;
and mixtures thereof;
and wherein the superdisintegrant is selected from the group consisting of sodium starch glycolate, croscarmellose sodium, crospovidon, low-substituted hydroxypropyl cellulose, soy non-starch polysaccharides, polacrilin potassium, calcium alginate, alginic acid, and mixtures thereof.

2. Method according claim 1, wherein the binder comprises a disaccharide.

3. Method according to claim 2, wherein the disaccharide comprises lactose.

4. Method according to claim 1, wherein the binder is microcrystalline cellulose.

5. Method according to any one of claims 1 - 4, wherein the temperature in steps a. - g. is kept between 10 °C - 60 °C.

6. Method according to any one of claims 1 - 5, wherein the wt./wt. ratio of binder to superdisintegrant is between 99/1 and 80/20.

7. Method according to any one of claims 1 - 6, wherein wt./wt. ratio of the solvent to binder is between 1/100 and 50/100.

8. Method according to any one of claim 1 - 7 wherein the solvent is selected from the group consisting of water, an organic solvent, and mixtures thereof.

9. Method according to claim 8, wherein the solvent is water.

10. Method according to any one of claims 1 - 9, wherein the co-processed excipient granules are further subjected to a milling and/or sieving step.

11. Method according to any one of claims 1 - 10, wherein the mixing zone further comprises at least one comb mixer element or screw mixer element.

12. Method according to any one of claims 1 - 11, wherein the screw speed of the extruder is kept constant, preferably at a rate of 100 - 1000 rpm.

13. Method according to any one of claims 1 - 11, wherein the angular velocity of the screw is set at a value between 1 - 42 rad/s.

14. Co-processed excipient granules obtainable by any one of claims 1 - 13.

15. Use of co-processed excipient granules obtained by the method of any one of claims 1 - 13 in a tableting process.

## Patentansprüche

1. Verfahren zum Herstellen co-verarbeiteter Hilfsstoffgranulate unter Verwendung eines corotierenden Doppelschneckenextruders, wobei der Doppelschneckenextruder zumindest eine Förderzone, eine Knetzone und eine Mischzone aufweist, wobei das Verfahren die folgenden Schritte umfasst:
a. Zuführen mindestens eines Bindemittels in die Förderzone des Extruders;
b. Fördern des Bindemittels in Richtung der Knetzone;
c. Zuführen eines Lösemittels an mindestens einer Stelle zwischen dem Anfang und dem Ende der Förderzone des Extruders, wodurch das Bindemittel befeuchtet wird;
d. Kneten des befeuchteten Bindemittels in der Knetzone des Extruders, um Bindemittelagglomerate zu bilden;
e. Fördern der gekneteten, befeuchteten Bindemittelagglomerate zu der Mischzone;
f. Zuführen eines oder mehrerer Supersprengmittel zu der Mischzone, wodurch Agglomerate von Bindemittel und dem einen oder den mehreren Supersprengmitteln gebildet werden;
g. Sammeln der Agglomerate von Bindemittel und Supersprengmittel aus dem Extruder; und
h. Trocknen der Agglomerate von Bindemittel und Supersprengmittel, um die co-verarbeiteten Hilfsstoffgranulate zu bilden;
wobei das Bindemittel ausgewählt ist aus der Gruppe bestehend aus
(i) Disacchariden;
(ii) Monosacchariden;
(iii) Polyolen;
(iv) Cellulosederivaten, die ausgewählt sind aus der Gruppe bestehend aus Cellulosefasern, mikrokristalliner Cellulose, Carboxymethylcellulose, Hydroxypropylmethylcellulose, Hydroxypropylcellulose und Gemischen davon;
(v) Stärken;
(vi) anorganischen Salzen;
und Gemischen davon;
und wobei das Supersprengmittel ausgewählt ist aus der Gruppe bestehend aus Natriumstärkeglycolat, Croscarmellose-Natrium, Crospovidon, niedrig substituierter Hydroxypropylcellulose, Soja-Nicht-Stärke-Polysacchariden, Polacrilin-Kalium, Calciumalginat, Alginsäure und Gemischen davon.

2. Verfahren nach Anspruch 1, wobei das Bindemittel ein Disaccharid umfasst.

3. Verfahren nach Anspruch 2, wobei das Disaccharid Lactose umfasst.

4. Verfahren nach Anspruch 1, wobei das Bindemittel mikrokristalline Cellulose ist.

5. Verfahren nach einem der Ansprüche 1 - 4, wobei die Temperatur in den Schritten a. - g. zwischen 10 °C - 60 °C gehalten wird.

6. Verfahren nach einem der Ansprüche 1 - 5, wobei das Gew./Gew.-Verhältnis von Bindemittel zu Supersprengmittel zwischen 99/1 und 80/20 liegt.

7. Verfahren nach einem der Ansprüche 1 - 6, wobei das Gew./Gew.-Verhältnis des Lösemittels zu dem Bindemittel zwischen 1/100 und 50/100 liegt.

8. Verfahren nach einem der Ansprüche 1 - 7, wobei das Lösemittel ausgewählt ist aus der Gruppe bestehend aus Wasser, einem organischen Lösemittel und Gemischen davon.

9. Verfahren nach Anspruch 8, wobei das Lösemittel Wasser ist.

10. Verfahren nach einem der Ansprüche 1 - 9, wobei die co-verarbeiteten Hilfsstoffgranulate ferner einem Mahl- und/oder Siebschritt unterzogen werden.

11. Verfahren nach einem der Ansprüche 1 - 10, wobei die Mischzone ferner mindestens ein Kammmischerelement oder Schneckenmischerelement umfasst.

12. Verfahren nach einem der Ansprüche 1 - 11, wobei die Schneckendrehzahl des Extruders konstant gehalten wird, vorzugsweise bei einer Geschwindigkeit von 100 - 1000 rpm.

13. Verfahren nach einem der Ansprüche 1 - 11, wobei die Winkelgeschwindigkeit der Schnecke auf einen Wert zwischen 1 - 42 rad/s eingestellt wird.

14. Co-verarbeitete Hilfsstoffgranulate, erhältlich durch einen der Ansprüche 1 - 13.

15. Verwendung durch das Verfahren nach einem der Ansprüche 1 - 13 erhaltener co-verarbeiteter Hilfsstoffgranulate in einem Tablettierungsprozess.

## Revendications

1. Procédé de préparation de granules d'excipient co-traités au moyen d'une extrudeuse à deux vis co-rotatives, l'extrudeuse à deux vis ayant au moins une zone de transport, une zone de malaxage et une zone de mélange, le procédé comprenant les étapes de
a. alimentation d'au moins un liant dans la zone de transport de l'extrudeuse ;
b. transport du liant vers la zone de malaxage ;
c. introduction d'un solvant dans au moins un point entre le début et la fin de la zone de transport de l'extrudeuse, mouillant ainsi le liant ;
d. malaxage du liant mouillé dans la zone de malaxage de l'extrudeuse pour former des agglomérats de liant ;
e. transport des agglomérats de liant malaxé et mouillé vers la zone de mélange ;
f. alimentation d'un ou plusieurs superdésintégrants dans la zone de mélange, formant ainsi des agglomérats de liant et de superdésintégrants ;
g. collecte des agglomérats de liant et de superdésintégrant de l'extrudeuse ; et
h. séchage des agglomérats de liant et de superdésintégrant pour former les granulés d'excipient cotraités ;
dans lequel le liant est choisi dans le groupe constitué par des
(i) disaccharides ;
(ii) monosaccharides ;
(iii) polyols ;
(iv) dérivés cellulosiques choisis dans le groupe constitué par les fibres de cellulose, la cellulose microcristalline, la carboxyméthylcellulose, l'hydroxypropylméthylcellulose, l'hydroxypropylcellulose, et leurs mélanges ;
(v) amidons ;
(vi) sels inorganiques ;
et leurs mélanges ;
et dans lequel le superdésintégrant est choisi dans le groupe constitué par le glycolate d'amidon sodique, la croscarmellose sodique, la crospovidone, l'hydroxypropylcellulose faiblement substituée, les polysaccharides non amidonés de soja, la polacriline de potassium, l'alginate de calcium, l'acide alginique et leurs mélanges.

2. Procédé selon la revendication 1, dans lequel le liant comprend un disaccharide.

3. Procédé selon la revendication 2, dans lequel le disaccharide comprend du lactose.

4. Procédé selon la revendication 1, dans lequel le liant est de la cellulose microcristalline.

5. Procédé selon l'une quelconque des revendications 1 à 4, dans lequel la température dans les étapes a. à g. est maintenue entre 10 °C et 60 °C.

6. Procédé selon l'une quelconque des revendications 1 à 5, dans lequel le rapport poids/poids de liant sur superdésintégrant est compris entre 99/1 et 80/20.

7. Procédé selon l'une quelconque des revendications 1 à 6, dans lequel le rapport poids/poids du solvant sur liant est compris entre 1/100 et 50/100.

8. Procédé selon l'une quelconque des revendications 1 à 7, dans lequel le solvant est choisi dans le groupe constitué par l'eau, un solvant organique et leurs mélanges.

9. Procédé selon la revendication 8, dans lequel le solvant est l'eau.

10. Procédé selon l'une quelconque des revendications 1 à 9, dans lequel les granules d'excipient co-traités sont en outre soumis à une étape de broyage et/ou de tamisage.

11. Procédé selon l'une quelconque des revendications 1 à 10, dans lequel la zone de mélange comprend en outre au moins un élément mélangeur en peigne ou un élément mélangeur à vis.

12. Procédé selon l'une quelconque des revendications 1 à 11, dans lequel la vitesse de la vis de l'extrudeuse est maintenue constante, de préférence à une vitesse de 100 à 1 000 tpm.

13. Procédé selon l'une quelconque des revendications 1 à 11, dans lequel la vitesse angulaire de la vis est réglée à une valeur comprise entre 1 et 42 rad/s.

14. Granulés d'excipient co-traités pouvant être obtenus selon l'une quelconque des revendications 1 à 13.

15. Utilisation de granules d'excipient co-traités obtenus par le procédé selon l'une quelconque des revendications 1 à 13 dans un procédé de mise en comprimé.
